# EUROPEAN PATENT APPLICATION

(11) **EP 3 766 504 A1**
(43) Date of publication of application: **20.01.2021**
(21) Application number: 19767271.0
(22) Date of filing: 13.03.2019
(51) Int. Cl.: A61K 33/06, A61K 9/08, A61K 9/10, A61K 31/198, A61K 47/12, A61K 47/18, A61K 47/22, A61K 47/44, A61P 3/02

(54) **TRANSFUSION PREPARATION**

(30) Priority: 13.03.2018 JP 2018045822
(71) Applicant: Otsuka Pharmaceutical Factory, Inc., Naruto-shi, Tokushima 772-8601 (JP)
(72) Inventor: TANI, Seiji, Naruto-shi, Tokushima 772-8601 (JP); FUJITA, Seiji, Naruto-shi, Tokushima 772-8601 (JP); NAKAI, Teru, Naruto-shi, Tokushima 772-8601 (JP); KIUCHI, Yasuhiro, Naruto-shi, Tokushima 772-8601 (JP); YAMANAKA, Miyuki, Naruto-shi, Tokushima 772-8601 (JP); HAYASHI, Yui, Naruto-shi, Tokushima 772-8601 (JP); KANNO, Hiroshi, Naruto-shi, Tokushima 772-8601 (JP); SARUWATARI, Yu, Naruto-shi, Tokushima 772-8601 (JP)
(74) Representative: Hoffmann Eitle
(86) International application number: PCT/JP2019/010195
(87) International publication number: WO 2019/176996

(57) **Abstract**

The present invention provides an infusion preparation that is inhi ited from generation of unwanted insolu le matter after mixing of two liquids of the infusion preparation in long-term storage. More spe ifi ally, the present invention provides an infusion preparation omprising two ham ers separated y a ommuni a ly opena le partition, a first ham er ontaining a first- ham er infusion omprising a fat emulsion and further omprising at least one mem er sele ted from the group onsisting of amino a ids that have a uffer a tion, divalent organi a ids, and trivalent organi a ids, a se ond ham er ontaining a se ond- ham er infusion omprising an amino a id and at least al ium as an ele trolyte, wherein a total on entration of the amino a ids that have a uffer a tion, divalent organi a ids, and trivalent organi a ids in the first- ham er infusion is 0.15 to 0.5 g/L, and a mixture of the first- and se ond- ham er infusions has a pH of 6.53 or less as measured 48 hours after the partition is ommuni a ly opened.

## Description

### Technical Field

The present invention relates to an infusion preparation comprising a fat, an amino acid, and an electrolyte. More specifically, the present invention provides an infusion preparation, which is an infusion preparation contained in a two-chamber container, wherein a first chamber contains a sugar and a fat emulsion, and a second chamber contains an amino acid and an electrolyte.

### Background Art

As an infusion preparation for parenteral nutrition (PN), there is proposed an infusion preparation (an intravenous nutrient infusion preparation) comprising two chambers separated by a communicably openable partition, wherein a first chamber contains a first-chamber infusion comprising a sugar and a fat emulsion, and a second chamber contains a second-chamber infusion comprising an amino acid and an electrolyte (Patent Literature (PTL) 1).

These infusion preparations are used, for example, for peripheral parenteral nutrition (PPN) and central parenteral nutrition (TPN) methods.

These infusion preparations are transported to facilities of use, such as hospitals, through distribution and sale after production, and are stored in the facilities until the infusion preparations are actually used clinically. These infusion preparations are required to be stable during the period from production to clinical use. The stability of an infusion preparation is usually ensured by testing and clarifying the period during which stability can be guaranteed, and setting the expiration date for use of the infusion preparation so as not to exceed the stability guarantee period.

### Citation List

### Patent Literature

PTL 1: WO2012/073891

### Summary of Invention

### Technical Problem

Through their own investigation, the present inventors found out that when an intravenous nutrient infusion preparation is left for a certain period of time after production, unwanted insoluble matter may be observed in a mixture of a first-chamber infusion and a second-chamber infusion after a partition between first and second chambers is opened to allow for communication.

An object of the present invention is to provide an infusion preparation that is inhibited from generation of unwanted insoluble matter after mixing two liquids of the infusion preparation in long-term storage.

### Solution to Problem

Through their own investigation, the present inventors elucidated the cause of generation of such unwanted insoluble matter after mixing two liquids. The inventors clarified that free fatty acids are formed in a first-chamber infusion during long-term storage, and unwanted insoluble matter is generated after mixing two liquids due to the presence of such free fatty acids. Based on this finding, the inventors repeated trial and error, and found that when a first-chamber infusion comprises at least one member selected from the group consisting of amino acids that have a buffer action, divalent organic acids, and trivalent organic acids in a concentration of 0.15 g/L or more, formation of free fatty acids in the first-chamber infusion during long-term storage can be inhibited. The inventors further found that if a mixture of first- and second-chamber infusions formed after a partition between first and second chambers is opened to allow for communication has a pH of 6.53 or less, generation of unwanted insoluble matter, which would occur due to the presence of such free fatty acids after mixing two liquids, can be inhibited.

The present invention has been accomplished with further improvements, and includes the following embodiments.

### Item 1.

An infusion preparation comprising two chambers separated by a communicably openable partition,
a first chamber containing a first-chamber infusion comprising a fat emulsion and further comprising at least one member selected from the group consisting of amino acids that have a buffer action, divalent organic acids, and trivalent organic acids,
a second chamber containing a second-chamber infusion comprising an amino acid and at least calcium as an electrolyte, wherein
a total concentration of the amino acids that have a buffer action, divalent organic acids, and trivalent organic acids in the first-chamber infusion is 0.15 to 0.5 g/L, and a mixture of the first- and second-chamber infusions has a pH of 6.53 or less as measured 48 hours after the partition is communicably opened.

### Item 2.

The infusion preparation according to Item 1, wherein the fat emulsion comprises an emulsifying agent, and the first-chamber infusion comprises the emulsifying agent in a concentration of 0.01 to 2 w/v%.

### Item 3.

The infusion preparation according to Item 1 or 2, wherein the mixture of the first- and second-chamber infusions after the partition is communicably opened contains Ca²⁺ in a concentration of 1 mEq/L or more.

### Item 4.

The infusion preparation according to any one of Items 1 to 3, wherein the second-chamber infusion further comprises at least citric acid as the electrolyte, and the second-chamber infusion has a citric acid concentration (mEq) equal to or more than the calcium concentration (mEq).

### Item 5.

The infusion preparation according to any one of Items 1 to 4, wherein the first-chamber infusion comprises at least histidine as the amino acids that have a buffer action. Item 6.

A mixture of the first- and second-chamber infusions of any one of Items 1 to 5 obtained by communicably opening the partition.

### Advantageous Effects of Invention

The infusion preparation of the present invention is inhibited from generation of unwanted insoluble matter after mixing two liquids in long-term storage. Therefore, the usable period of the infusion preparation can be set longer.

The present invention is described below in more detail.

The present invention provides an infusion preparation comprising two chambers separated by a communicably openable partition, a first chamber containing a first-chamber infusion comprising a fat emulsion, a second chamber containing a second-chamber infusion comprising an amino acid and an electrolyte.

### First-Chamber Infusion

The first-chamber infusion used in the present invention comprises a fat emulsion and further comprises at least one member selected from the group consisting of amino acids that have a buffer action, divalent organic acids, and trivalent organic acids.

The fat emulsion incorporated in the first-chamber infusion is an oil-in-water emulsion that is produced by dispersing an oil and/or fat in water by using an emulsifying agent. The fat emulsion can be produced according to a usual method. For example, the fat emulsion can be produced by adding an oil and/or fat, and an emulsifying agent to water, stirring the mixture to prepare a crude emulsion, and emulsifying the crude emulsion by a conventional method, such as a high-pressure emulsification method.

The oil and/or fat is preferably edible oil. Specific examples include vegetable oils (e.g., soybean oil, olive oil, cottonseed oil, safflower oil, corn oil, coconut oil, and perilla oil); fish oils (e.g., sardine oil); medium-chain fatty acid triglycerides (C₈₋₁₀ fatty acid triglycerides) (e.g., product names: PANACET (produced by NOF Corporation), ODO (produced by Nisshin Oil Mills, Ltd.), COCONARD (produced by Kao Corporation), and Miglyol (produced by Mitsuba Trading Co., Ltd.)); synthetic triglycerides (e.g., 2-linoleoyl-1,3-dioctanoyl glycerol (8L8) and 2-linoleoyl-1,3-didecanoyl-glycerol (10L10)); and the like. Such oils and fats may be used singly or in a combination of two or more.

The emulsifying agent may be selected from, for example, various pharmaceutically acceptable emulsifying agents. Specific examples include egg yolk phospholipid (egg yolk lecithin), hydrogenated egg yolk phospholipid, soybean phospholipid (soybean lecithin), hydrogenated soybean phospholipid, nonionic surfactants, and the like. Such emulsifying agents may be used singly or in a combination of two or more.

The oil and/or fat is particularly preferably soybean oil. The emulsifying agent is particularly preferably egg yolk phospholipid (egg yolk lecithin). Lecithin, such as egg yolk lecithin, is preferable because it can also act as a phosphorus source as described below.

Insofar as an oil-in-water fat emulsion is produced, the amounts of oil and/or fat and emulsifying agent used to prepare the fat emulsion are not particularly limited. The oil and/or fat is preferably used in such an amount as to achieve a concentration of about 0.5 to 6 w/v%, and more preferably about 1 to 5 w/v% in the obtained fat emulsion. Furthermore, the emulsifying agent is preferably used in such an amount as to achieve a concentration of about 0.01 to 2 w/v%, more preferably about 0.1 to 1.5 w/v%, and still more preferably about 0.2 to 1.5 w/v% in the obtained fat emulsion. The concentration of the emulsifying agent in the first-chamber infusion within this range can, together with other features of the present invention, inhibit the formation of free fatty acids during long-term storage, thereby inhibiting the generation of unwanted insoluble matter after mixing the two liquids in long-term storage.

One specific example of a particularly preferable method for producing the fat emulsion according to the present invention is described below. Specifically, an oil and/or fat and an emulsifying agent are added to water, and at least one member selected from glycerol and glucose is added thereto. The mixture is then stirred to prepare a crude emulsion. Subsequently, the crude emulsion is emulsified by a conventional method, such as a high-pressure emulsification method. The high-pressure emulsification method may be carried out, for example, by passing the crude emulsion through an emulsifier, such as a Manton Gaulin homogenizer, at a rate of 20 to 700 kg/cm² about 2 to 50 times, and preferably 3 to 20 times. In this method, insofar as glycerol and/or glucose is present during the emulsification, how and when they are added are not limited. For example, glycerol and/or glucose may be added to the crude emulsion prepared by using an oil and/or fat and an emulsifying agent, and the resulting crude emulsion may be emulsified. The obtained fat emulsion contains glycerol and/or glucose in a concentration of preferably about 5 to 70 w/v%, and more preferably about 10 to 30 w/v%.

The first-chamber infusion further comprises at least one member selected from the group consisting of amino acids that have a buffer action, divalent organic acids, and trivalent organic acids. In the first-chamber infusion, the total concentration of the amino acids that have a buffer action, divalent organic acids, and trivalent organic acids is 0.15 to 0.5 g/L. The total concentration of the amino acids that have a buffer action is calculated based on the amount of these amino acids as free amino acids. Without being bound by theory, the study of the present inventors suggests that free fatty acids are formed by decomposition of an oil and/or fat or an emulsifier during long-term storage, and it has also been revealed that the degree of the formation by decomposition depends on pH. Further, a reduction in pH accompanied by the formation of free fatty acids presumably further promotes the formation of free fatty acids. Therefore, to inhibit the generation of unwanted insoluble matter after mixing of the two liquids, it appeared to be important to maintain the pH of the first-chamber infusion within an optimal range. To achieve this, it is considered to be important to enhance the buffer action of the first-chamber infusion at a pH within the optimal range to thus maintain the pH of the first-chamber infusion within this optimal range. The optimal pH range that can prevent an oil and/or fat and an emulsifier from being decomposed to form free fatty acids etc. is preferably 4.5 to 6.5, more preferably 5.0 to 6.5, and still more preferably 5.5 to 6.5.

The amino acids that have a buffer action are not particularly limited insofar as they are capable of enhancing the buffer action of the first-chamber infusion at the pH range in which an oil and/or fat and an emulsifier are prevented from being decomposed to form free fatty acids etc. to thus allow the pH of the first-chamber infusion to be within the above range. Examples include histidine, lysine, arginine, and the like. The presence of the amino acids that have a buffer action can prevent an oil and/or fat and an emulsifying agent from being decomposed to form free fatty acids etc., and suppress a reduction of the pH of the second-chamber infusion over time. Such amino acids that have a buffer action are preferably L-histidine and L-lysine, and more preferably L-histidine. The amino acids that have a buffer action furthermore preferably consist substantially only of L-histidine or consist of only L-histidine. Histidine also functions as a pH adjuster.

The divalent organic acids and trivalent organic acids are not particularly limited insofar as they are capable of enhancing the buffer action of the first-chamber infusion at the pH range in which an oil and/or fat and an emulsifier are prevented from being decomposed to form free fatty acids etc. to thus allow the pH of the first-chamber infusion to be within the above range. Examples include citric acid, succinic acid, malic acid, tartaric acid, and the like. The presence of the divalent organic acids or trivalent organic acids can prevent an oil and/or fat and an emulsifying agent from being decomposed to form free fatty acids etc., and suppress a reduction of the pH of the second-chamber infusion over time. Citric acid, succinic acid, malic acid, and tartaric acid also function as a pH adjuster.

When the first-chamber infusion comprises at least one member selected from the group consisting of amino acids that have a buffer action, divalent organic acids, and trivalent organic acids in a total concentration of 0.15 g/L or more, the formation of free fatty acids in the first-chamber infusion is inhibited during long-term storage, whereby the generation of unwanted insoluble matter is inhibited after mixing of the two liquids in long-term storage. To achieve this effect, the first-chamber infusion preferably comprises the at least one member selected from the group consisting of amino acids that have a buffer action, divalent organic acids, and trivalent organic acids in a total concentration of 0.2 g/L or more. In the first-chamber infusion, the upper limit of the total concentration of the at least one member selected from the group consisting of amino acids that have a buffer action, divalent organic acids, and trivalent organic acids is for example, 0.6 g/L, and preferably 0.5 g/L. The total concentration of the at least one member selected from the group consisting of amino acids that have a buffer action, divalent organic acids, and trivalent organic acids, contained in the first-chamber infusion is preferably within this range, in view of an actual performance as a pharmaceutical additive and prevention of discoloration when a reducing sugar, such as glucose, is incorporated. To summarize the above, the first-chamber infusion comprises the at least one member selected from the group consisting of amino acids that have a buffer action, divalent organic acids, and trivalent organic acids in a total concentration of preferably 0.15 to 0.6 g/L, and more preferably 0.2 to 0.3 g/L.

Further, various known additives that can be added to fat emulsions may be also optionally incorporated. Examples of such additives include pH adjusters. The pH adjusters may be those that are known to be used in infusion preparations. For example, organic acids and amino acids may be used, in addition to acids such as hydrochloric acid, and alkalis such as sodium hydroxide and potassium hydroxide. Examples of organic acids include acetic acid, lactic acid, and the like. Examples of amino acids include L-lysine and the like. Among these, oil-soluble materials may be mixed in advance with oily components of the emulsion, while water-soluble materials may be mixed with water for injection, or may be added to the aqueous phase of the obtained fat emulsion. The amounts of additives can be suitably determined, and may be, for example, the same as conventionally known amounts.

The first-chamber infusion comprises fat emulsion in a concentration of, for example, 0.5 to 6 w/v%, preferably 1 to 5 w/v%, and more preferably 2 to 5 w/v%, based on the amount of oils and fats. In the infusion preparation of the present invention, the mixture of the first- and second-chamber infusions comprises fat emulsion in a concentration of, for example, 0.25 to 6 w/v%, preferably 0.5 to 3 w/v%, and more preferably 1 to 2.5 w/v%, based on the amount of oils and fats.

The first-chamber infusion preferably has a pH of 4.5 to 6.5, more preferably 5.0 to 6.5, and still more preferably 5.5 to 6.5. When the pH is within this range, it is possible to prevent an oil/and fat and an emulsifier from being decomposed to form free fatty acids etc., and to stabilize the fat emulsion and vitamin B₁ in the first-chamber infusion. The pH of the first-chamber infusion can be adjusted by using a pH adjuster. L-histidine, L-arginine, malic acid, citric acid, succinic acid, tartaric acid, etc., mentioned earlier, may be used as a pH adjuster. It is also possible to use hydrochloric acid, acetic acid, lactic acid, sodium hydroxide, potassium hydroxide, etc. as a pH adjuster.

The first-chamber infusion preferably comprises a sugar. Examples of sugars that can be incorporated include reducing sugars such as glucose, fructose, and maltose; nonreducing sugars such as xylitol, sorbitol, and glycerol; and the like. Among these sugars, a reducing sugar is preferably incorporated in the first-chamber infusion, and glucose is more preferably incorporated from the viewpoint of blood glucose level management etc. Such sugars may be used singly or in a combination of two or more.

The first-chamber infusion comprises a sugar in a concentration of preferably 70 to 150 g/L. Further, the mixture of the first- and second-chamber infusions obtained by communicably opening the partition preferably has a sugar concentration of 50 to 100 g/L, and more preferably 50 to 75 g/L.

To prevent the onset of acidosis during infusion therapy, vitamin B₁ is preferably incorporated in the first-chamber infusion. Examples of vitamin B₁ that can be incorporated in the first-chamber infusion include thiamine chloride hydrochloride, thiamine mononitrate, prosultiamine, octotiamine, and the like.

The first-chamber infusion comprises vitamin B₁ in a concentration of, for example, 1.5 to 10 mg/L, and preferably 2 to 8 mg/L, based on the thiamine amount. In the infusion preparation of the present invention, the mixture of the first- and second-chamber infusions obtained by communicably opening the partition preferably has a vitamin B₁ concentration of 1 to 6 mg/L, and more preferably 1.5 to 4 mg/L, based on the thiamine amount.

In view of further enhancing the stability of vitamin B₁, the first-chamber infusion preferably has a titratable acidity of 10 or less. The titratable acidity refers to an amount (mL) of a 0.1 mol/L sodium hydroxide aqueous solution required to neutralize 100 ml of a solution to pH 7.4.

Distilled water for injection can be typically used as a solvent of the first-chamber infusion.

In the infusion preparation of the present invention, the fluid volume of the first-chamber infusion is suitably determined according to, for example, the total fluid volume of the infusion preparation or the fluid volume of the second-chamber infusion.

The first-chamber infusion may be substantially free of potassium. The phrase "substantially free of potassium" means that no potassium-containing compounds are added. Further, the first-chamber infusion is preferably substantially free of calcium. The phrase "substantially free of calcium" means that no calcium-containing compounds are added.

The first-chamber infusion has a relative osmotic pressure of 2.0 to 4.0, and preferably about 2.0 to 3.5. The relative osmotic pressure as used herein refers to a ratio relative to the osmotic pressure of physiological saline (i.e., a relative ratio with the osmotic pressure of physiological saline defined as 1). The relative osmotic pressure of the infusion refers to a ratio relative to the osmotic pressure of physiological saline unless otherwise specified.

### Second-Chamber Infusion

The second-chamber infusion used in the present invention comprises an amino acid and calcium as an electrolyte.

The amino acids incorporated in the second-chamber infusion are not limited as long as they can be incorporated in amino acid infusions for nutritional supplementation for the body. In the present invention, the amino acids are typically used in the form of a free amino acid. However, amino acids in the form of a pharmaceutically acceptable salt, an ester, a N-acyl derivative, or a dipeptide may also be used. Specific examples of free amino acids that can be incorporated in the second-chamber infusion include L-leucine, L-isoleucine, L-valine, L-lysine, L-threonine, L-tryptophan, L-methionine, L-phenylalanine, L-cysteine, L-tyrosine, L-arginine, L-histidine, L-alanine, L-proline, L-serine, glycine, L-aspartic acid, L-glutamic acid, and the like. Specific examples of amino acid salts include inorganic acid salts such as L-arginine hydrochloride, L-cysteine hydrochloride, L-glutamic acid hydrochloride, L-histidine hydrochloride, and L-lysine hydrochloride; organic acid salts such as L-lysine acetate and L-lysine malate; and the like. Specific examples of amino acid esters include L-tyrosine methyl ester, L-methionine methyl ester, L-methionine ethyl ester, and the like. Specific examples of N-acyl amino acids include N-acetyl-L-cysteine, N-acetyl-L-tryptophan, N-acetyl-L-proline, and the like. Specific examples of amino acid dipeptides include L-tyrosyl-L-tyrosine, L-alanyl-L-tyrosine, L-arginyl-L-tyrosine, L-tyrosyl-L-arginine, and the like. In particular, L-cysteine is preferably incorporated in the form of acetylcysteine, in view of stability. Such amino acids may be used singly, but are preferably used in a combination of two or more from the viewpoint of nutritional supplementation. For example, it is preferable to incorporate at least all of the essential amino acids (i.e., 9 types of amino acids: L-leucine, L-isoleucine, L-valine, L-lysine, L-threonine, L-tryptophan, L-methionine, L-phenylalanine, and L-histidine).

The second-chamber infusion comprises amino acids in a concentration of preferably 40 to 120 g/L, and more preferably 50 to 100 g/L, based on the total amount of free amino acids. In the infusion preparation of the present invention, the mixture of the first- and second-chamber infusions preferably has an amino acid concentration of 10 to 50 g/L, and more preferably 20 to 40 g/L, based on the total amount of free amino acids.

Preferable combinations of amino acids and their proportions in the second-chamber infusion are, for example, as follows in terms of free amino acids: L-leucine: 5 to 15 g/L; L-isoleucine: 3 to 9 g/L; L-valine: 3 to 9 g/L; L-lysine: 3 to 12 g/L; L-threonine: 1.2 to 6 g/L; L-tryptophan: 0.3 to 3 g/L; L-methionine: 0.6 to 4.8 g/L; L-phenylalanine: 1.8 to 9 g/L; L-cysteine: 0.1 to 1.8 g/L; L-tyrosine: 0.06 to 1.2 g/L; L-arginine: 3 to 12 g/L; L-histidine: 1.2 to 6 g/L; L-alanine: 3 to 9 g/L; L-proline: 1.2 to 6 g/L; L-serine: 0.6 to 4.2 g/L; glycine: 1.2 to 6 g/L; L-aspartic acid: 0.12 to 1.8 g/L; and L-glutamic acid: 0.12 to 1.8 g/L.

In the infusion preparation of the present invention, the mixture of the first- and second-chamber infusions preferably contains amino acids in the following concentrations in terms of free amino acids: L-leucine: 3 to 9 g/L; L-isoleucine: 1.5 to 4.5 g/L; L-valine: 1.5 to 4.5 g/L; L-lysine: 1.5 to 5 g/L; L-threonine: 0.6 to 3 g/L; L-tryptophan: 0.15 to 1.5 g/L; L-methionine: 0.3 to 2.4 g/L; L-phenylalanine: 0.85 to 4.5 g/L; L-cysteine: 0.03 to 0.9 g/L; L-tyrosine: 0.03 to 0.6 g/L; L-arginine: 1.5 to 5 g/L; L-histidine: 0.6 to 3 g/L; L-alanine: 1.5 to 4.5 g/L; L-proline: 0.6 to 3 g/L; L-serine: 0.3 to 2.1 g/L; glycine: 0.6 to 3 g/L; L-aspartic acid: 0.06 to 0.9 g/L; and L-glutamic acid: 0.06 to 0.9 g/L.

The electrolyte incorporated in the second-chamber infusion is an electrolyte that is used in the infusion field and serves as an active ingredient rather than an additive or the like. More specifically, the electrolyte is one contained in a body fluid (e.g., blood or intracellular fluid) (body fluid electrolyte), and can be referred to as a "physiologically important electrolyte." Specific examples of such electrolytes include potassium, calcium, sodium, magnesium, phosphorus, zinc, chlorine, and the like. In the infusion preparation of the present invention, the first-chamber infusion preferably does not comprise such an electrolyte. Potassium, in particular, is usually incorporated in both infusions of a two-chamber infusion preparation in order to avoid the risk of administering a high concentration of potassium; however, in the infusion preparation of the present invention, potassium is incorporated only in the second-chamber infusion.

Examples of calcium sources include calcium salts, such as calcium gluconate, calcium chloride, calcium glycerophosphate, calcium lactate, calcium pantothenate, calcium acetate, and the like. Calcium salts may be in the form of a hydrate (e.g., calcium gluconate hydrate). The second-chamber infusion comprises calcium in a concentration of preferably 1 or more, and more preferably 6 to 12 mEq/L. In the infusion preparation of the present invention, the mixture of first- and second-chamber infusions has a calcium concentration of 1 or more, preferably 1 or more and 9 mEq/L or less, and more preferably 3 to 6 mEq/L.

Examples of potassium sources include potassium chloride, potassium acetate, potassium citrate, potassium glycerophosphate, potassium sulfate, potassium lactate, and the like. Among these, potassium glycerophosphate is preferable because it also acts as a phosphorus source. Such potassium sources may be in the form of a hydrate. The second-chamber infusion comprises potassium in a concentration of preferably 40 mEq/L or less (more preferably 25 to 40 mEq/L). In the infusion preparation of the present invention, the mixture of the first- and second-chamber infusions has a potassium concentration of preferably 16 mEq/L or more, more preferably 16 to 25 mEq/L, and still more preferably 16 to 20 mEq/L.

Examples of sodium sources include sodium salts such as sodium chloride, sodium lactate, sodium acetate, sodium sulfate, sodium gly erophosphate, sodium itrate, and sodium la tate. When the infusion preparation of the present invention ontains phosphorus and al ium and/or magnesium, it is prefera le to use sodium itrate as (part of) the sodium sour es in order to prevent pre ipitation of these elements. Sodium sour es may e in the form of a hydrate. The se ond- ham er infusion omprises sodium in a on entration of, for example, 50 to 100 mEq/L, and prefera ly 40 to 80 mEq/L. In the infusion preparation of the present invention, the mixture of the first- and se ond- ham er infusions has a sodium on entration of, for example, 25 to 50 mEq/L, and prefera ly 30 to 40 mEq/L.

Examples of magnesium sour es in lude magnesium sulfate, magnesium hloride, magnesium a etate, and the like. Magnesium sour es may e in the form of a hydrate. The se ond-ham er infusion omprises magnesium in a on entration of prefera ly 1 to 20 mEq/L, and more prefera ly 5 to 15 mEq/L. In the infusion preparation of the present invention, the mixture of the first- and se ond- ham er infusions prefera ly has a magnesium on entration of 0.5 to 10 mEq/L, and more prefera ly 2 to 6 mEq/L.

Prefera le examples of phosphorus sour es are organi salts, su h as sodium gly erophosphate and potassium gly erophosphate, sin e the use of an inorgani salt as a phosphorus sour e may result in pre ipitation of al ium phosphate or magnesium phosphate. When le ithin is used as an emulsifying agent in the first ham er, the le ithin also a ts as a phosphorus sour e. When phosphorus from the le ithin is present in a suffi ient amount, it is unne essary to add phosphorus to the se ond ham er, whi h is prefera le sin e no pre ipitation of al ium phosphate et . would o ur. The se ond- ham er infusion ontains phosphorus in a on entration of, for example, 0 to 20 mmol/L. In the infusion preparation of the present invention, the mixture of the first- and se ond- ham er infusions prefera ly has a phosphorus on entration of 1 to 20 mmol/L, and more prefera ly 5 to 10 mmol/L.

Examples of zin sour es in lude zin sulfate, zin hloride, and the like. Zin sour es may e in the form of a hydrate. The se ond- ham er infusion omprises zin in a on entration of, for example, 2.5 to 15 µmol/L. In the infusion preparation of the present invention, the mixture of the first- and se ond- ham er infusions prefera ly has a zin on entration of 1.5 to 9 µmol/L.

Examples of hlorine sour es in lude sodium hloride, potassium hloride, magnesium hloride, al ium hloride, and the like. The se ond- ham er infusion omprises hlorine in a on entration of, for example, 50 to 100 mEq/L, and prefera ly 40 to 80 mEq/L. In the infusion preparation of the present invention, the mixture of the first- and se ond- ham er infusions prefera ly has a hlorine on entration of 25 to 60 mEq/L, and more prefera ly 30 to 40 mEq/L.

The pH of the se ond- ham er infusion may e optionally adjusted to 6.0 to 6.8, and prefera ly 6.2 to 6.7, y using a pH adjuster. The pH adjuster here may e the same as those mentioned a ove for the first- ham er infusion. When the se ond- ham er infusion has a pH within this range, amino a ids that are prone to undergo hemi al hanges, su h as L- ysteine and L-glutami a id, an e sta ilized. Furthermore, the pH of the mixture o tained y mixing the first- ham er infusion and se ond- ham er infusion an e maintained within the optimum range mentioned elow.

In orporation of at least one diproti a id and/or tri asi a id sele ted from the group onsisting of itri a id, mali a id, and su ini a id in the se ond- ham er infusion an inhi it the pre ipitation of al ium phosphate that o urs due to the formation of phosphori a id, whi h is an impurity or a de omposition produ t of gly erophosphori a id. In parti ular, itri a id is preferred as the diproti a id and/or tri asi a id. The total on entration (mEq) of diproti a ids and tri asi a ids in the se ond- ham er infusion is prefera ly equal to or more than the al ium on entration (mEq).

As a solvent in the se ond- ham er infusion as well, distilled water for inje tion an e typi ally used.

In the infusion preparation of the present invention, the se ond- ham er infusion has a relative osmoti pressure of a out 2.0 to 3.5, and prefera ly a out 2.0 to 3.0.

Further, the infusion preparation of the present invention may optionally ontain a sta ilizer. Examples of sta ilizers that an e in orporated in the infusion preparation of the present invention in lude sulfites su h as sodium isulfite. To avoid de omposition of vitamin B₁ ontained in the first- ham er infusion, sulfite is in orporated in the se ond-ham er infusion. The se ond- ham er infusion omprises sulfite in a on entration of, for example, 20 to 100 mg/L.

In addition to vitamin B₁, various types of vitamins an e added to the infusion preparation of the present invention. Various types of vitamins an e sta ly added to the infusion preparation in the two- ham er ontainer without the need to pla e the infusion preparation in a three- or four-ham er ontainer. This is one of the features of the infusion preparation of the present invention. Vitamins are lassified into water-solu le vitamins and fat-solu le vitamins . In the infusion preparation of the present invention, a fat-solu le vitamin is added to the first- ham er infusion. Further, a water-solu le vitamin may e added to either the first- or se ond-ham er infusion. However, as des ri ed a ove, vitamin B₁ is added to the first- ham er infusion.

Examples of water-solu le vitamins added to the infusion preparation of the present invention in lude B- omplex vitamins and vitamin C. In addition to vitamin B₁ (thiamine), examples of B- omplex vitamins in lude vitamin B₂ (ri oflavin), vitamin B₃ (nia in) , vitamin B₅ (pantotheni a id), vitamin B₆, vitamin B₇ (iotin), vitamin B₉ (foli a id), vitamin B₁₂ ( yano o alamin), and the like. Further, examples of fat-solu le vitamins in lude vitamin A, vitamin D (in parti ular, hole al iferol), vitamin E, vitamin K, and the like.

Vitamin C (as or i a id), when used, an e added to the first- or se ond- ham er infusion, or oth. However, it is prefera ly added to the se ond- ham er infusion. When vitamin C is added to the se ond- ham er infusion, the on entration of vitamin C in the se ond- ham er infusion is, for example, 50 to 500 mg/L, and prefera ly 100 to 400 mg/L. Further, in the infusion preparation of the present invention, the vitamin C on entration in the mixture of the first- and se ond- ham er infusions is prefera ly set to satisfy the following ranges: for example, 25 to 250 mg/L, prefera ly 50 to 200 mg/L, and more prefera ly 40 to 100 mg/L.

Usa le vitamin B₂ in ludes ri oflavin, ri oflavin sodium phosphate, flavin mononu leotide, and the like. Vitamin B₂, when used, an e added to the first- or se ond- ham er infusion, or oth. However, vitamin B₂ and foli a id are prefera ly pla ed in different ham ers in order to prevent the desta ilization of foli a id aused y a rea tion etween vitamin B₂ and foli a id. For example, when foli a id is added to the first- ham er infusion, vitamin B₂ is prefera ly added to the se ond- ham er infusion. When vitamin B₂ is added to the se ond- ham er infusion, the on entration of vitamin B₂ in the se ond- ham er infusion is, for example, 2.5 to 15 mg/L, and prefera ly 4 to 8 mg/L, on the ri oflavin asis. Further, in the infusion preparation of the present invention, the vitamin B₂ on entration in the mixture of the first- and se ond- ham er infusions is prefera ly set to satisfy the following ranges: for example, 0.5 to 10 mg/L, and prefera ly 0.5 to 3 mg/L, on the ri oflavin asis.

Usa le vitamin B₆ in lude pyridoxine, salts of pyridoxine, su h as pyridoxine hydro hloride, and the like. Vitamin B₆, when used, an e added to the first- or se ond-ham er infusion, or oth. However, vitamin B₆ e omes very unsta le to light when it is present with vitamin B₂. Therefore, vitamin B₆ is prefera ly added to the infusion to whi h vitamin B₂ is not added. When vitamin B₆ is added to the first- ham er infusion, the on entration of vitamin B₆ in the first- ham er infusion is, for example, 2 to 10 mg/L, and prefera ly 2.5 to 6 mg/L, on the pyridoxine asis. Further, in the infusion preparation of the present invention, the vitamin B₆ on entration in the mixture of the first- and se ond- ham er infusions is prefera ly set to satisfy the following ranges: for example, 1 to 10 mg/L, and prefera ly 1.5 to 4.5 mg/L, on the pyridoxine asis.

Foli a id, when used, an e added to the first- or se ond- ham er infusion, or oth; however, it is prefera ly added to the first- ham er infusion. When foli a id is added to the first- ham er infusion, the on entration of foli a id in the first- ham er infusion is, for example, 0.1 to 0.8 mg/L, and prefera ly 0.2 to 0.6 mg/L. Further, in the infusion preparation of the present invention, the foli a id on entration in the mixture of the first- and se ond- ham er infusions is prefera ly set to satisfy the following ranges: for example, 0.1 to 0.7 mg/L, and prefera ly 0.2 to 0.4 mg/L.

Usa le vitamin B₁₂ in ludes yano o alamin, hydroxo o alamin a etate, methyl o alamin, and the like. Vitamin B₁₂, when used, an e added to the first- or se ond- ham er infusion, or oth; however, it is prefera ly added to the first-ham er infusion when the se ond ham er ontains sulfite. When vitamin B₁₂ is added to the first- ham er infusion, the on entration of vitamin B₁₂ in the first- ham er infusion is, for example, 2 to 10 µg/L, and prefera ly 2.5 to 6 µg/L. Further, in the infusion preparation of the present invention, the vitamin B₁₂ on entration in the mixture of the first- and se ond- ham er infusions is prefera ly set to satisfy the following ranges: for example, 0.5 to 10 mg/L, and prefera ly 0.5 to 3 mg/L.

As nia in, for example, ni otinamide is prefera ly used. Nia in, when used, an e added to the first- or se ond-ham er infusion, or oth; however, it is prefera ly added to the se ond- ham er infusion. When nia in is added to the se ond-ham er infusion, the on entration of nia in in the se ond-ham er infusion is, for example, 10 to 100 mg/L, and prefera ly 20 to 50 mg/L. Further, in the infusion preparation of the present invention, the nia in on entration in the mixture of the first- and se ond- ham er infusions is prefera ly set to satisfy the following ranges: for example, 5 to 50 mg/L, and prefera ly 5 to 25 mg/L.

As pantotheni a id, panthenol is prefera ly used. Pantotheni a id, when used, an e added to the first- or se ond- ham er infusion, or oth. When pantotheni a id is added to the first- or se ond- ham er infusion, the on entration of pantotheni a id is, for example, 5 to 30 mg/L, and prefera ly 10 to 20 mg/L, on the panthenol asis. Further, in the infusion preparation of the present invention, the panthenol on entration in the mixture of the first- and se ond- ham er infusions is set to satisfy the following ranges: for example, 2.5 to 15 mg/L, and prefera ly 5 to 10 mg/L.

Biotin, when used, an e added to the first- or se ond- ham er infusion, or oth; however, it is prefera ly added to the se ond- ham er infusion. When iotin is added to the se ond- ham er infusion, the on entration of iotin in the se ond- ham er infusion is, for example, 10 to 100 µg/L, and prefera ly 20 to 80 µg/L. Further, in the infusion preparation of the present invention, the iotin on entration in the mixture of the first- and se ond- ham er infusions is prefera ly set to satisfy the following ranges: for example, 1 to 50 µg/L, and prefera ly 10 to 40 µg/L.

As vitamin A, retinol palmitate is prefera ly used. Further, vitamin A oil formed y dissolving retinol palmitate in oil an also e used. Vitamin A, whi h is fat-solu le, is added to the first- ham er infusion. The on entration of vitamin A in the first- ham er infusion is, for example, 1,000 to 5,000 IU/L, and prefera ly 2,000 to 4,000 IU/L. Further, in the infusion preparation of the present invention, the vitamin A on entration in the mixture of the first- and se ond- ham er infusions is prefera ly set to satisfy the following ranges: for example, 500 to 2,500 IU/L, and prefera ly 1,000 to 2,000 IU/L. "IU" stands for international unit. It is also alled vitamin A unit.

As vitamin D, hole al iferol (vitamin D₃) is prefera ly used. Vitamin D, whi h is fat-solu le, is added to the first- ham er infusion. The on entration of vitamin D in the first- ham er infusion is, for example, 2 to 10 µg/L, and prefera ly 2.5 to 6 µg/L. Further, in the infusion preparation of the present invention, the vitamin D on entration in the mixture of the first- and se ond- ham er infusions is prefera ly set to satisfy the following ranges: for example, 0.5 to 10 µg/L, and prefera ly 0.5 to 3 µg/L.

As vitamin E, to opherol a etate is prefera ly used. Vitamin E, whi h is fat-solu le, is added to the first- ham er infusion. The on entration of vitamin E in the first- ham er infusion is, for example, 2 to 50 mg/L, and prefera ly 5 to 20 mg/L. Further, in the infusion preparation of the present invention, the vitamin E on entration in the mixture of the first- and se ond- ham er infusions is prefera ly set to satisfy the following ranges: for example, 1 to 25 mg/L, and prefera ly 2.5 to 10 mg/L.

As vitamin K, phytonadione (vitamin K₁) is prefera ly used. Vitamin K, whi h is fat-solu le, is added to the first-ham er infusion. The on entration of vitamin K in the first-ham er infusion is, for example, 50 to 2,500 µg/L, and prefera ly 80 to 2,000 µg/L. Further, in the infusion preparation of the present invention, the vitamin K on entration in the mixture of the first- and se ond- ham er infusions is prefera ly set to satisfy the following ranges: for example, 20 to 1,200 µg/L, and prefera ly 30 to 1,000 µg/L.

A prefera le example of the a tive ingredients ontained in the first- and se ond- ham er infusion ompositions is shown elow. At least one mem er sele ted from the group onsisting of amino a ids that have a uffer a tion, divalent organi a ids, and trivalent organi a ids is separately added to the first ham er.

### First-Cham er Infusion

Purified soy ean oil: 10-50 g/L
Glu ose: 70-150 g/L
Thiamine hloride hydro hloride: 3-10 mg/L
Pyridoxine hydro hloride: 3-7 mg/L
Cyano o alamin: 2.5-5 µg/L
Foli a id: 0.2-0.5 mg/L
Vitamin A oil: 2,000-4,000 IU/L
Chole al iferol: 2.5-5 µg/L
To opherol a etate: 5-20 mg/L
Phytonadione: 80-2,000 µg/L
Panthenol: 10-20 mg/L

### Se ond-Cham er Infusion

L-leu ine: 5-15 g/L
L-isoleu ine: 3-9 g/L
L-valine: 3-9 g/L
L-lysine hydro hloride: 3.5-15 g/L
L-threonine: 1.2-6 g/L
L-tryptophan: 0.3-3 g/L
L-methionine: 0.6-4.8 g/L
A etyl ysteine: 0.13-2.4 g/L
L-phenylalanine: 1.8-9 g/L
L-tyrosine: 0.06-1.2 g/L
L-arginine: 3-12 g/L
L-histidine: 1.2-6 g/L
L-alanine: 3-9 g/L
L-proline: 1.2-6 g/L
L-serine: 0.6-4.2 g/L
Gly ine: 1.2-6 g/L
L-asparati a id: 0.12-1.8 g/L
L-glutami a id: 0.12-1.8 g/L
Sodium: 40-80 mEq/L
Potassium: 25-40 mEq/L
Cal ium: 6-12 mEq/L
Magnesium: 5-15 mEq/L
Chlorine: 40-80 mEq/L
Phosphorus: 0-20 mmoL/L
Zin : 2.5-15 µmol/L
Ri oflavin sodium phosphate: 5-10 mg/L
As or i a id: 0.1-0.4 g/L
Biotin: 20-80 µg/L
Ni otinamide: 20-50 mg/L

Both the first- and se ond- ham er infusions an e produ ed y a known method for produ ing infusions. For example, the first- and se ond- ham er infusions an e produ ed y dissolving ea h of the infusion omponents des ri ed a ove in distilled water for inje tion. Fat-solu le omponents are prefera ly used, for example, during emulsifi ation as des ri ed a ove.

### Mixture of the First- and Se ond-Cham er Infusions

At the time of use of the infusion preparation of the present invention, the first- ham er infusion and the se ond-ham er infusion are mixed. The mixture of the first- and se ond-ham er infusions has a pH of 6.53 or less, and prefera ly 6.4 or less. The pH of the mixture within this range, together with the features of the present invention, an a hieve inhi ition of the generation of unwanted insolu le matter after mixing of the two liquids in long-term storage. More spe ifi ally, first, the other features of the present invention a hieve inhi ition of the formation of free fatty a ids in the first- ham er infusion during long-term storage. Further, y adjusting the pH of the mixture within the a ove range, the generation of unwanted insolu le matter in the mixture due to the presen e of free fatty a ids is inhi ited.

A ordingly, to ultimately inhi it the generation of unwanted insolu le matter, it is important to effe tively om ine other features of the present invention, in parti ular, a feature that the first- ham er infusion omprises at least one mem er sele ted from the group onsisting of amino a ids that have a uffer a tion, divalent organi a ids, and trivalent organi a ids, su h as histidine, in a total on entration of 0.15 g/L or more; and a feature that the mixture has a pH of 6.53 or less. In this sense, it is suffi ient when the first- ham er infusion omprises at least one mem er sele ted from the group onsisting of amino a ids that have a uffer a tion, divalent organi a ids, and trivalent organi a ids in a total on entration of 0.15 g/L or more, and the mixture has a pH of 6.53 or less. It is prefera le that (1) the first- ham er infusion omprises at least one mem er sele ted from the group onsisting of amino a ids that have a uffer a tion, divalent organi a ids, and trivalent organi a ids in a total on entration of 0.3 g/L or more, and the mixture has a pH of 6.53 or less; or (2) the first- ham er infusion omprises at least one mem er sele ted from the group onsisting of amino a ids that have a uffer a tion, divalent organi a ids, and trivalent organi a ids in a total on entration of 0.2 g/L or more, and the mixture has a pH of 6.4 or less.

The present inventors found that free fatty a ids generate unwanted insolu le matter in the mixture spe ifi ally when free fatty a ids form a salt with Ca²⁺. Therefore, to ultimately inhi it the generation of unwanted insolu le matter, the Ca₂₊ on entration is prefera ly redu ed in the mixture of the first- and se ond- ham er infusions, and the Ca²⁺ on entration is prefera ly 3 to 6 mEq/L in the mixture of the first- and se ond-ham er infusions.

The infusion preparation of the present invention has a titrata le a idity of prefera ly 1 to 10 and a relative osmoti pressure of prefera ly 2 to 3.

Further, in the infusion preparation of the present invention, the volume ratio etween the first- ham er infusion and the se ond- ham er infusion is suita ly determined a ording to, for example, the fluid volumes of the first- and se ond-ham er infusions des ri ed a ove. In view of the sta ility of ea h omponent and the osmoti pressure setting in ea h ham er, the volume ratio (first- ham er infusion:se ond- ham er infusion) is, for example, 3:2-3:5.

Further, the alorifi value of the mixture is prefera ly 450 to 750 k al/L, and more prefera ly 500 to 650 k al/L. In this alorifi value, the per entage of fat is prefera ly 40% or less, and more prefera ly 20 to 40%. Further, in this alorifi value, the per entages of sugar, fat, and amino a id are prefera ly as follows: 40 to 60% sugar, 20 to 40% fat, and 10 to 30% amino a id; and more prefera ly, 45 to 55% sugar, 25 to 35% fat, and 15 to 25% amino a id.

An approximate alorifi value of ea h omponent an e determined y multiplying the amount (g) y 4 for sugar, y 9 for fat, and y 4 for amino a id. Spe ifi ally, the alorifi value of sugar is a out 4 k al/g, the alorifi value of fat is a out 9 k al/g, and the alorifi value of amino a id is a out 4 k al/g. An approximate alorifi value an e determined ased on this information. The alorifi value of the mixture des ri ed a ove is ased on the value al ulated a ordingly.

A prefera le example of the omposition of ea h omponent in the mixture is shown elow.

**Ta le 1**

| | | |
|---|---|---|
| Ele trolytes | Na | 35 mEq |
| | K | 20 mEq |
| | Mg | 5 mEq |
| | Ca | 5 mEq |
| | Cl | 35 mEq |
| | P | 10 mmol |
| | Zn | 5 µmol |
| Sugar | Glu ose | 75 g |
| Fat | Purified soy ean oil | 20 g |
| Amino a id | Amino a id | 30 g |
| Vitamins | Thiamine hloride hydro hloride | 1.9 mg |
| | Ri oflavin sodium phosphate | 2.3 mg |
| | Pyridoxine hydro hloride | 2.45 mg |
| | Cyano o alamin | 2.5 µg |
| | Ni otinamide | 20 mg |
| | Panthenol | 7 mg |
| | Foli a id | 0.2 mg |
| | Biotin | 30 µg |
| | As or i a id | 50 mg |
| | Vitamin A oil | 1,650 IU |
| | Chole al iferol | 2.5 µg |
| | To opherol a etate | 5 mg |
| | Phytonadione | 1 mg |
| Additives | Egg yolk le ithin | 2.4 g |
| | L-histidine | 0.2 g |

### Infusion Preparation Usage Form

The infusion preparation of the present invention is used in order to manage the nutrition of a perioperative patient when the patient has mild hypoproteinemia or mild malnutrition due to inadequate oral intake or when the patient is in the invasive phase. In parti ular, the infusion preparation is suita ly used to manage the nutrition of a patient having diffi ulty re eiving oral nutritional support in the postoperative period or due to a digestive disorder and the like (prefera ly, a patient who has undergone gastri rese tion surgery). The infusion preparation of the present invention is administered to a patient for 1 to 14 days after surgery, and prefera ly 1 to 3 days after surgery. The nutritional status of the patient an there y e maintained in a healthy state. The dose and the dosing rate an e suita ly determined in view of ea h patient's symptoms, age, and the like. In parti ular, when the infusion preparation of the present invention is used, the infusion preparation an maintain, y itself, the nutritional status of the patient in a healthy state for the duration of administration.

The infusion preparation of the present invention is prefera ly administered into a peripheral vein. In other words, the infusion preparation of the present invention is prefera ly an infusion preparation for peripheral intravenous administration. In general, administration of an infusion that has overly high osmoti pressure into a peripheral vein an ause vas ular pain or phle itis. However, there is no su h risk when the infusion preparation of the present invention is used. Therefore, the effe t of the infusion preparation of the present invention is suita ly demonstrated when the infusion preparation is administered into a peripheral vein.

### Infusion Container

The ontainer in whi h the first- ham er infusion and the se ond- ham er infusion are pla ed is not parti ularly limited insofar as the ontainer has two ham ers that are inter ommuni a le. Examples in lude two- ham er ontainers (infusion ags) in whi h the ham ers are separated y a partition wall that an e ommuni a ly opened, su h as ones in whi h a partition wall is formed y an easily peela le seal (Japanese Unexamined Patent Pu li ation No. H2-4671, Japanese Unexamined Utility Model Pu li ation No. H5-5138, and the like), ones in whi h a partition wall is formed y lipping the spa e etween the ham ers (Japanese Unexamined Patent Pu li ation No. S63-309263 and the like), and ones in whi h various ommuni ating means that an open the partition wall is provided to the partition wall (Japanese Examined Patent Pu li ation No. S63-20550 and the like). Of these, an infusion ag in whi h the partition wall is formed y an easily peela le seal is prefera le e ause it is suita le for mass produ tion and the ham ers an e easily rought into ommuni ation. Further, various gas-permea le plasti s ommonly used for medi al ontainers are used as materials of the a ove ontainer. Examples in lude flexi le plasti s, su h as polyethylene, polypropylene, polyvinyl hloride, rosslinked ethylene-vinyl a etate opolymer, ethylene-α-olefin opolymer, lends of su h polymers, and laminates omprising su h polymers.

The first- and se ond- ham er infusions an e filled and pa kaged in the ontainer y a onventional method. For example, the ham ers are filled with the infusions under an inert gas atmosphere, sealed, and sterilized y heat.

Heat sterilization an e performed y a known method, su h as high-pressure steam sterilization or hot water shower sterilization. If ne essary, the heat sterilization an e arried out in an inert gas atmosphere su h as ar on dioxide or nitrogen. The infusion preparation of the present invention does not generate unwanted insolu le matter even when sterilized in high-pressure steam at 116 to 121°C.

Further, the first- and se ond- ham er infusions ontained in the ontainer are prefera ly pa kaged together with a deoxidant in an oxygen arrier exterior ag in order to relia ly prevent deterioration, oxidation, et . In parti ular, when an infusion ag in whi h the partition wall is formed y an easily peela le seal is used as a ontainer, the infusion ag is prefera ly pa kaged in su h a manner that the infusion ag is folded, for example, in half, at the easily peela le seal portion to prevent the partition wall from eing ommuni a ly opened y external pressure. Further, for example, filling and pa kaging may e optionally performed with an inert gas.

Commonly or widely used films, sheets, and the like formed from various materials an e used as materials of the oxygen arrier exterior ag suita le for the pa kage. Spe ifi examples in lude ethylene vinylal ohol opolymer, polyvinylidene hloride, polya rylonitrile, polyvinyl al ohol, polyamide, polyester, and the like. Examples also in lude films and sheets formed from materials omprising at least one of these materials.

Usa le deoxidants in lude various known deoxidants, su h as those omprising, as an a tive ingredient, ferri hydroxide, ferri oxide, iron ar ide, or other iron ompounds, and those omprising low-mole ular-weight phenol and a tivated ar on. Examples of produ t names of typi al ommer ial produ ts in lude Ageless (produ ed y Mitsu ishi Gas Chemi al), Moduran (produ ed y Nippon Kayaku), Se ur (produ ed y Nippon Soda), Tamotsu (produ ed y Oji Kako), Keepit (produ ed y Dren y), and the like.

### Examples

The present invention is des ri ed in more detail elow with referen e to Examples. The invention, however, is not limited to the following Examples.

### Produ tion of Infusion Preparations

### 1. Produ tion of First-Cham er Infusions 1 to 4

Purified soy ean oil, purified egg yolk le ithin, and glu ose in the amounts shown in Ta le 2 were added to water. The resulting mixture was su je ted to rude emulsifi ation using a homomixer. The resulting rude emulsion was then su je ted to fine emulsifi ation using a high-pressure emulsifier (Manton-Gaulin). Further, L-histidine in the amounts shown in Ta le 2 and water were added thereto to make the total volume of ea h emulsion 300 mL. Further, hydro hlori a id was used to adjust the pH. The first- ham er infusions thus o tained had a relative osmoti pressure of 2.9 and a titrata le a idity of 1.

**Table 2**

| | Formulation 1 | Formulation 2 | Formulation 3 | Formulation 4 |
|---|---|---|---|---|
| Purified soy ean oil | 10 g | | | |
| Glu ose | 37.5 g | | | |
| Purified egg yolk le ithin | 1.2 g | | | |
| L-histidine | 0.03 g | 0.06 g | 0.09 g | 0.15 g |
| pH | 6.3 | 6.3 | 6.3 | 6.3 |

### 2. Formulation of Se ond-Cham er Infusions A to D

Amino a ids, ele trolytes, and a sta ilizer (sodium hydrogen sulfite) in the amounts shown in Ta le 3 were dissolved in distilled water for inje tion to prepare an amino a id ele trolyte solution. Further, the pH of the ele trolyte solution was adjusted to the levels shown in Ta le 3 with gla ial a eti a id, and the total volume of ea h solution was adjusted to 250 mL, giving se ond- ham er infusions. The se ond- ham er infusions thus o tained had a relative osmoti pressure of 2.7. The se ond-ham er infusions had a itri a id on entration of 12.68 mEq/L and a al ium on entration of 10.00 mEq/L.

**Table 3**

| | Formulation A | Formulation B | Formulation C |
|---|---|---|---|
| L-leu ine | 2.100 g | | |
| L-isoleu ine | 1.200 g | | |
| L-valine | 1.200 g | | |
| L-lysine hydro hloride | 1.965 g | | |
| L-threonine | 0.855 g | | |
| L-tryptophan | 0.300 g | | |
| L-methionine | 0.585 g | | |
| A etyl ysteine | 0.202 g | | |
| L-phenylalanine | 1.050 g | | |
| L-tyrosine | 0.075 g | | |
| L-arginine | 1.575 g | | |
| L-histidine | 0.750 g | | |
| L-alanine | 1.200 g | | |
| L-proline | 0.750 g | | |
| L-serine | 0.450 g | | |
| Gl y ine | 0.885 g | | |
| L-asparti a id | 0.150 g | | |
| L-glutami a id | 0.150 g | | |
| Sodium hloride | 0.215 g | | |
| Potassium hloride | 0.220 g | | |
| Sodium itrate hydrate | 0.311 g | | |
| Sodium L-la tate solution (60%)(as sodium L-la tate) | 1.967 g | | |
| | (1.180 g) | | |
| 50% potassium gly erophosphate solution (as potassium gly erophosphate) | 1.750 g | | |
| | (0.875 g) | | |
| Cal ium glu onate hydrate | 0.561 g | | |
| Magnesium sulfate hydrate | 0.309 g | | |
| Zin sulfate hydrate | 0.72 mg | | |
| Sodium isulfite | 12.5 mg | | |
| pH | 6.5 | 6.7 | 6.9 |

### 3. Filling and Pa kaging

Using two- ham er polyethylene ontainers with the ham ers eing partitioned y an easily peela le seal, formulations 1 to 4 were pla ed as the first- ham er infusion in the lower ham ers of the ontainers, whereas formulations A to C were pla ed as the se ond- ham er infusion in the upper ham ers of the ontainers. After the atmosphere in the va ant spa e of ea h ham er was repla ed with nitrogen gas and ea h ontainer was sealed, high-pressure steam sterilization was performed at a temperature of 116°C for 26 minutes. Ea h ontainer was then folded at the easily peela le seal portion, and en losed with a deoxidant in an exterior ag made of gas arrier film, thus o taining an infusion preparation. Three preparations of the same infusion were prepared for ea h type.

The infusion preparations o tained a ove, ontaining formulations 1 to 4 as the first- ham er infusion and formulation A as the se ond- ham er infusion, were stored at 60°C/75% RH for 3 weeks, and then the first- ham er infusion and se ond- ham er infusion were mixed. Whether unwanted insolu le matter was generated was onfirmed immediately after mixing, 26 hours after mixing, and 48 hours after mixing. The pH was measured 48 hours after mixing. Ta le 4 shows the results.

Whether free fatty a ids were formed was visually evaluated a ording to the following riteria.
A: No unwanted insolu le matter was o served on the inner surfa e of the ontainer.
B: White unwanted insolu le matter was o served on the inner surfa e of the ontainer.

**Ta le 4**

| Upper chamber: second-chamber infusion (pH) | Lower chamber: first -chamber infusion (amount of L-histidine added) | Round of tests | Confirmation on generation of unwanted insoluble matter | | | pH |
|---|---|---|---|---|---|---|
| | | | Measurement time | | | |
| | | | Immediately after mixing | 26 hours after mixing | 48 hours after mixing | |
| Formulation A (pH 6.5) | Formulation 1 (0.1 g/L) | 1st | A | B | B | 6.40 |
| | | 2nd | A | B | B | 6.39 |
| | | 3rd | A | B | B | 6.40 |
| | Formulation 2 (0.2 g/L) | 1st | A | A | A | 6.41 |
| | | 2nd | A | A | A | 6.40 |
| | | 3rd | A | A | A | 6.41 |
| | Formulation 3 (0.3 g/L) | 1st | A | A | A | 6.39 |
| | | 2nd | A | A | A | 6.40 |
| | | 3rd | A | A | A | 6.39 |
| | Formulation 4 (0.5 g/L) | 1st | A | A | A | 6.37 |
| | | 2nd | A | A | A | 6.37 |
| | | 3rd | A | A | A | 6.38 |

When the infusion preparation omprised formulation 1 ontaining L-histidine in a on entration of 0.1 g/L as the first- ham er infusion, white unwanted insolu le matter started to e o served on the inner surfa e of the ontainers ontaining the preparation 26 hours after mixing.

The infusion preparations o tained a ove, ea h ontaining one of formulations 1 to 4 as the first- ham er infusion and ontaining formulation B as the se ond- ham er infusion, were stored at 60°C/75% RH for 3 weeks, and then the first- ham er infusion and the se ond- ham er infusion were mixed. Whether unwanted insolu le matter was generated was onfirmed immediately after mixing, 26 hours after mixing, and 48 hours after mixing. The pH was measured 48 hours after mixing. Ta le 5 shows the results.

Whether free fatty a ids were formed was onfirmed in the same manner as des ri ed a ove.

**Ta le 5**

| Upper chamber: second-chamber infusion (pH) | Lower chamber: first -chamber infusion(amount of L-histidine added) | Round of tests | Confirmation on generation of unwanted insoluble matter | | | pH |
|---|---|---|---|---|---|---|
| | | | Measurement time | | | |
| | | | Immediately after mixing | 26 hours after mixing | 48 hours after mixing | |
| Formulation B (pH 6.7) | Formulation 1 (0.03 g/L) | 1st | A | B | B | 6.53 |
| | | 2nd | A | B | B | 6.53 |
| | | 3rd | A | B | B | 6.53 |
| | Formulation 2 (0.03 g/L) | 1st | A | B | B | 6.54 |
| | | 2nd | A | B | B | 6.55 |
| | | 3rd | A | B | B | 6.54 |
| | Formulation 3 (0.03 g/L) | 1st | A | A | A | 6.53 |
| | | 2nd | A | A | A | 6.53 |
| | | 3rd | A | A | A | 6.53 |
| | Formulation 4 (0.03 g/L) | 1st | A | A | A | 6.51 |
| | | 2nd | A | A | A | 6.52 |
| | | 3rd | A | A | A | 6.52 |

When the infusion preparation omprised formulation 1 ontaining L-histidine in a on entration of 0.1 g/L as the first- ham er infusion, white unwanted insolu le matter started to e o served on the inner surfa e of the ontainers ontaining the preparation 26 hours after mixing. Further, when the infusion preparation omprised formulation 2 and formulation B and had a pH of 6.54 or 6.55 after mixing, white unwanted insolu le matter started to e o served on the inner surfa e of the ontainers ontaining the mixture of formulation 2 and formulation B 26 hours after mixing.

The infusion preparations o tained a ove, ea h ontaining one of formulations 1 to 4 as the first- ham er infusion and formulation C as the se ond- ham er infusion, were stored at 60°C/75% RH for 3 weeks, and then the first- ham er infusion and the se ond- ham er infusion were mixed. Whether unwanted insolu le matter was generated was onfirmed immediately after mixing, 26 hours after mixing, and 48 hours after mixing. The pH was measured 48 hours after mixing. Ta le 6 shows the results.

Whether free fatty a ids were formed was onfirmed in the same manner as des ri ed a ove.

**Table 6**

| Upper chamber: second-chamber infusion (pH) | Lower chamber: first-chamber infusion (amount of L-histidine added) | Round of tests | Confirmation on generation of unwanted insoluble matter | | | pH |
|---|---|---|---|---|---|---|
| | | | Measurement time | | | |
| | | | Immediately after mixing | 26 hours after mixing | 48 hours after mixing | |
| Formulation C (pH 6.9) | Formulation 1 (0.03 g/300 mL) | 1st | A | B | B | 6.66 |
| | | 2nd | A | B | B | 6.68 |
| | | 3rd | A | B | B | 6.68 |
| | Formulation 2 (0.06 g/300 mL) | 1st | A | B | B | 6.67 |
| | | 2nd | A | B | B | 6.68 |
| | | 3rd | A | B | B | 6.68 |
| | Formulation 3 (0.09 g/300 mL) | 1st | A | B | B | 6.68 |
| | | 2nd | A | B | B | 6.68 |
| | | 3rd | A | B | B | 6.68 |
| | Formulation 4 (0.15 g/300 mL) | 1st | A | B | B | 6.65 |
| | | 2nd | A | B | B | 6.65 |
| | | 3rd | A | B | B | 6.65 |

Forty-eight hours after mixing, all of the infusion preparations ontaining formulation C having a pH of 6.9 as the se ond- ham er infusion had a pH higher than 6.53, no matter whi h of formulations 1 to 4 was used as the first- ham er infusion. Further, white unwanted insolu le matter started to e o served on the inner surfa e of the ontainers 26 hours after mixing.

## Claims

1. An infusion preparation omprising two ham ers separated y a ommuni a ly opena le partition,
a first ham er ontaining a first- ham er infusion omprising a fat emulsion and further omprising at least one mem er sele ted from the group onsisting of amino a ids that have a uffer a tion, divalent organi a ids, and trivalent organi a ids,
a se ond ham er ontaining a se ond- ham er infusion omprising an amino a id and at least al ium as an ele trolyte, wherein a total on entration of the amino a ids that have a uffer a tion, divalent organi a ids, and trivalent organi a ids in the first- ham er infusion is 0.15 to 0.5 g/L, and a mixture of the first- and se ond- ham er infusions has a pH of 6.53 or less as measured 48 hours after the partition is ommuni a ly opened.

2. The infusion preparation a ording to laim 1, wherein the fat emulsion omprises an emulsifying agent, and the first-ham er infusion omprises the emulsifying agent in a on entration of 0.01 to 2 w/v%.

3. The infusion preparation a ording to laim 1 or 2, wherein the mixture of the first- and se ond- ham er infusions after the partition is ommuni a ly opened ontains Ca²⁺ in a on entration of 1 mEq/L or more.

4. The infusion preparation a ording to any one of laims 1 to 3, wherein the se ond- ham er infusion further omprises at least itri a id as the ele trolyte, and the se ond- ham er infusion has a itri a id on entration (mEq) equal to or more than the al ium on entration (mEq).

5. The infusion preparation a ording to any one of laims 1 to 4, wherein the first- ham er infusion omprises at least histidine as the amino a ids that have a uffer a tion.

6. A mixture of the first- and se ond- ham er infusions of any one of laims 1 to 5 o tained y ommuni a ly opening the partition.
